# EUROPÄISCHE PATENTSCHRIFT

(11) **EP 0 204 089 B1**
(45) Veröffentlichungstag und Bekanntmachung des Hinweises auf die Patenterteilung: **09.06.1993**
(21) Anmeldenummer: 86103724.0
(22) Anmeldetag: 19.03.1986
(51) Int. Cl.: G05B 19/42

(54) **Verfahren zur Herstellung eines räumlichen Modells**
Method to create a three-dimensional model
Procédé pour la création d'un modèle à trois dimensions

(30) Priorität: 23.03.1985 DE 3510639
(43) Veröffentlichungstag der Anmeldung: 10.12.1986
(73) Patentinhaber: MDC Medical Diagnostic Computing GmbH, D-24106 Kiel (DE)
(72) Erfinder: Meyer, Wolfgang, Dr. med., D-2000 Hamburg 50 (DE)
(74) Vertreter: Heldt, Gert, Dr. Dipl.-Ing.

(56) Entgegenhaltungen:
- DE-A- 3 437 483
- US-A- 3 932 923
- US-A- 4 393 450
- PATENT ABSTRACTS OF JAPAN, Band 9, Nr. 275 (M-426)[1998], 2. November 1985;& JP-A-60 118 399

## Beschreibung

Die Erfindung betrifft ein Verfahren zur Herstellung eines räumlichen Modells von von außen nicht zugänglichen Strukturen mit Hilfe computer-tomographischer Teilschnitte, die auf vorgegebenen Ebenen in einer vorgegebenen Reihenfolge durch die Strukturen gelegt werden und deren Begrenzung auf einen Modellwerkstoff übertragen werden.

Ein derartiges Verfahren ist bekanntgeworden aus einem Aufsatz "Ein neues Verfahren zur Herstellung alloplastischer Spezialimplantate für den Becken-Teilersatz" der in der Zeitschrift "Fortschritte der Medizin", 97. Jahrgang, (1979) Nr. 16, S. 781-783, veröffentlicht worden ist. In diesem Aufsatz wird ein Verfahren beschrieben, wie mit Hilfe der Computer-Tomographie ein Modell eines Beckenknochens hergestellt werden kann, ohne daß dieser von außen unmittelbar erreichbar ist. Zu diesem Zwecke werden Kunststoff-Platten einer Dicke von etwa 15 mm verwendet, von denen jede einzelne entsprechend einem bestimmten Querschnitt durch den Beckenknochen gestaltet wird. Dieser Querschnitt wird mit Hilfe von Röntgenstrahlen abgetastet. Diese Abtastung erfolgt in Abständen von jeweils 15 mm. Die gewonnenen Querschnitte werden auf die Kunststoffplatten, insbesondere Styropor-Platten, übertragen. Die auf diese Weise gebildeten einzelnen Profile werden schrittweise, beispielsweise durch Klebetechnik, zu dem räumlichen Modell zusammengefügt. Entsprechend der Dicke der Platte besitzt ein auf diese Weise hergestelltes Modell eine treppenförmige Begrenzungsfläche, die jedoch im Sinne der Kenntnis des abzubildenden Körperteils durch Abheben der einzelnen Treppenstufen eingeebnet werden können. Dieser Modellbau erfordert ein gutes räumliches Vorstellungsvermögen und ein hohes Maß an handwerklichem Geschick. Ein solches Verfahren hat deshalb auch den Nachteil, daß es zeitaufwendig, teuer und teilweise auch ungenau ist.

Darüberhinaus ist aus der US-A-3 932 923 ein Verfahren zur Herstellung eines dreidimensionalen Modells bekanntgeworden, bei dem eine Oberfläche mechanisch abgetastet wird und die so gewonnenen Werte über einen Computer zur Herstellung von ebenen Elementen genutzt werden. Die einzelnen Elemente entsprechen in ihren Abmessungen einem bestimmten Querschnitt und werden anschließend, nachdem sie hergestellt worden sind, beispielsweise durch Verkleben zu einem räumlichen Modell des abgetasteten Knochens zusammengefügt.

Dieses Verfahren hat den entscheidenden Nachteil, daß es für von außen nicht zugängliche Strukturen aufgrund seiner mechanischen Abtastung nicht geeignet ist. Ein weiterer Nachteil ist die Herstellung mit Hilfe von ebenen Platten, durch deren paßgenaues Aneinanderfügen das räumliche Modell nur mittelbar entsteht.

Darüberhinaus ist eine Vorrichtung zur Herstellung eines dreidimensionalen Modells bekannt, bei der als Schneidwerkzeug ein Teilbereich eines Schneidedrahtes dient, der zwischen zwei Punkten fest eingespannt ist (US-A-4 393 450). Diese Vorrichtung läßt nicht erkennen, daß sie auch von einem Computertomographen gesteuert werden kann. Gerade in der computertomographischen Aufnahme bestimmter Begrenzungslinien, die auf unmittelbare Weise nicht erfaßt werden können, und deren Umsetzung zur Steuerung von Schneidwerkzeugen liegt die wesentliche Problematik, die bisher zu einer befriedigenden Lösung nicht geführt hat. Obgleich ein erheblicher Bedarf besteht, Modelle von lebenden Gewebeteilen herzustellen, um beispielsweise eine detaillierte Operationsplanung durchführen zu können, wurde dieses Problem bisher von der einschlägigen Fachwelt noch nicht gelöst.

Aufgabe der vorliegenden Erfindung ist es daher, das Verfahren der einleitend genannten Art so zu verbessern, daß es zur schnellen und kostengünstigen Herstellung sehr genauer Modelle benutzt werden kann.

Diese Aufgabe wird erfindungsgemäß dadurch gelöst, daß Begrenzungslinien der computer-tomographischen Teilschnitte in räumliche Koordinaten elektronisch zur Steuerung mindestens eines Bearbeitungswerkzeuges umgesetzt werden, wobei das Bearbeitungswerkzeug im Bereich zwischen zwei Teilschnitten liegender Abstände mit Hilfe interpolierter Werte gesteuert wird, die vom Rechner aufgrund der auf den Teilschnitten liegenden Koordinaten berechnet werden, und ein Werkstück unmittelbar aus einem Block des Modellwerkstoffes hergestellt wird.

Mit diesem Verfahren können die computer-tomographischen Teilschnitte optimal ausgewertet werden. Eine Umsetzung dieser Teilschnitte in ein entsprechendes Bild des Teilschnittes kann erspart werden, da die Koordinaten der Begrenzungspunkte unmittelbar in elektrische Impulse umgesetzt werden können, die der Steuerung des Bearbeitungswerkzeuges dienen. Durch Ermittlung von Werten, die zwischen zwei benachbarten Teilschnitten liegen, aus einer Interpolation von Koordinaten, die die einander benachbarte Ebene begrenzen, entfällt die bisher nachteilige stufige Oberfläche bzw. deren nachträgliche Einebnung. Durch die Herstellung des Modells bzw. Werkstückes unmittelbar aus einem Block des Modellwerkstoffes entfällt zugleich das Verkleben der einzelnen Schichten und die damit verbundenen Ungenauigkeiten.

Gemäß einer bevorzugten Ausführungsform der Erfindung wird das Bearbeitungswerkzeug auf gedachten Ebenen, die in ihrer Lage einem jeweiligen Teilabschnitt entsprechen, an einem zur Herstellung des Modells geeigneten Block des Modellwerkstoffes entlanggeführt. Dabei wird das Modell unmittelbar aus einem Block hergestellt, innerhalb dessen lediglich gedachte Ebenen im Bereich der computer-tomographischen Teilschnitte verlaufen. Je nach der Entfernung, die zwischen zwei benachbarten Teilschnitten liegen, entsteht auch bei diesem Modell eine mehr oder minder in Stufen verlaufende Oberfläche, die jedoch im Rahmen einer Feinbearbeitung eingeebnet werden kann. Diese Feinbearbeitung erfolgt mit Hilfe der Interpolation von Koordinaten, die einander benachbarte Ebenen begrenzen.

Gemäß einer weiteren bevorzugten Ausführungsform der Erfindung werden die räumlichen Koordinaten eines jeden Teilschnittes in einen Computer eingelesen und in diesem gespeichert. Diese zusätzliche Speicherung der einzelnen Koordinaten hat den Vorteil, daß sie ohne Schwierigkeiten aus dem Computer wieder entnommen werden können, um sie zur Herstellung weiterer Modelle zu benutzen. Darüberhinaus ist es jedoch auch möglich, die eingelesenen Daten mit den Daten einer späteren Computer-tomographischen Aufnahme zu vergleichen. Auf diese Weise können auch Unterschiede von sehr geringen Abmessungen erkannt werden, die möglicherweise bei der Betrachtung des Modells nicht auffallen würden.

Weitere Einzelheiten der Erfindung ergeben sich aus der nachfolgenden ausführlichen Beschreibung und den beigefügten Zeichnungen, in denen eine bevorzugte Ausführungsform der Erfindung beispielsweise veranschaulicht ist.

In den Zeichnungen zeigen:

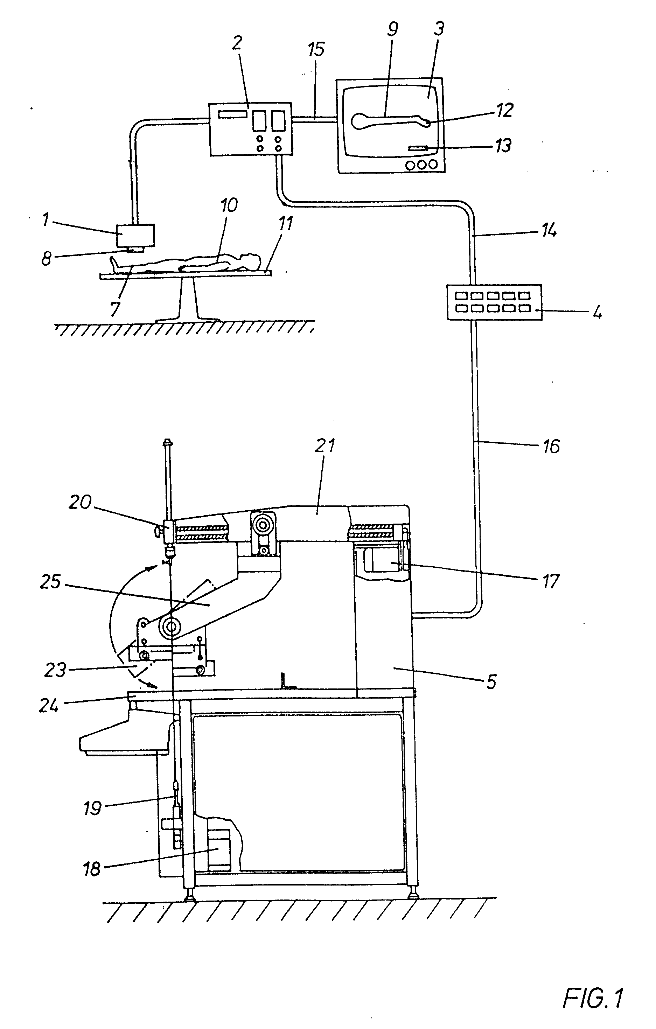

- Fig. 1:: eine schematische Darstellung einer Anlage zur Durchführung des Verfahrens
und

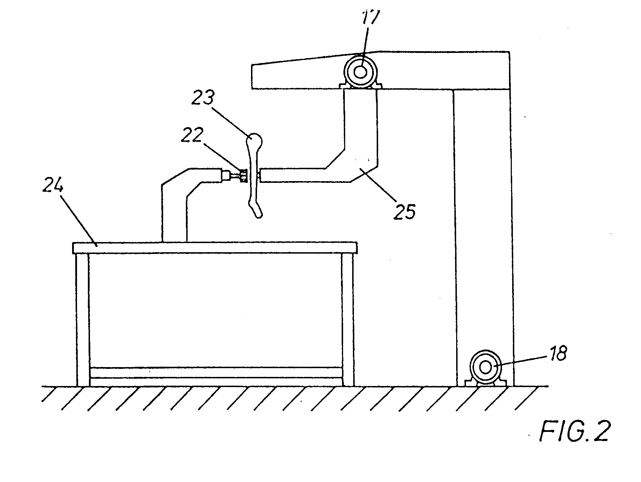

- Fig. 2:: eine schematische Darstellung einer Maschine zur Herstellung des räumlichen Modells.

Das erfindungsgemäße Verfahren wird zweckmäßigerweise durchgeführt mit einer Anlage, die im wesentlichen besteht aus einem Computertomographen (1), einem Computer (2), einem Monitor (3), einem Bedienungspult (4) sowie einer Bearbeitungsmaschine (5). Der Computertomograph (1) ist mit dem Computer (2) verbunden, sodaß die vom Computertomographen (1) aufgenommenen elektrischen Impulse in den Computer (2) eingespeist werden. Diese elektrischen Impulse entsprechen Begrenzungslinien eines Körperteils (7), auf das der Computertomograph (1) mit seiner Aufnahmeeinheit (8) gerichtet ist. Bei diesem Körperteil (7) kann es sich beispielsweise um einen Oberschenkelknochen eines Menschen (10) handeln, der auf einem Behandlungtisch (11) im Einflußbereich der Aufnahmeeinheit (8) liegt.

In den Computer (2) eingespeiste elektrische Impulse werden gespeichert, beispielsweise in einer Speichereinheit des Computers (2), auf einer Magnetplatte oder einem Magnetband. Aus dieser Speichereinheit kann ein vom Computertomographen (1) durch das Körperteil (7) gelegter Teilschnitt (12) auf dem Monitor (3) abgebildet werden. Dieser Monitor (3) kann mit dem Computer (2) im Dialogsystem geschaltet sein, so daß die Begrenzungslinien (9) des Teilschnitts (12) von einem Cursor umfahren werden können. Der Cursor (13) wird vom Bedienungspult (4) gesteuert, das mit dem Computer (2) über eine Verbindungsleitung (14) verbunden ist. Dieser ist mit dem Monitor (3) über eine Verbindungsleitung (15) verbunden.

Darüberhinaus ist das Bedienungspult (4) über eine weitere Verbindungsleitung (16) mit der Bearbeitungsmaschine (5) verbunden. Diese Bearbeitungsmaschine (5) wird entsprechend dem auf dem Monitor (3) jeweils abgebildeten Teilschnitt (12) gesteuert. Zu diesem Zwecke besitzt die Bearbeitungsmaschine Motore (17, 18) für Bewegungen in Y-Richtung einerseits und in Z-Richtung andererseits. Ein weiterer Motor für Bewegungen des Schlittens in X-Richtung ist vorhanden, in der Zeichnung jedoch nicht dargestellt. Darüberhinaus ist ein weiterer Antrieb für Rotationsbewegungen vorgesehen, aber ebenfalls nicht dargestellt.

In der Bearbeitungsmaschine (5) wird ein Bearbeitungswerkzeug (19) am vorderen Ende (20) eines Trägerarmes (21) geführt. Bei diesem Bearbeitungswerkzeug (7) kann es sich beispielsweise um einen Draht, einen Stift oder eine Schlaufe handeln, die jeweils auf eine Temperatur von beispielsweise 180 Grad C erwärmt werden. In diesem erhitzten Zustand ist es in der Lage, einen aus Kunststoff, beispielsweise Styropor bestehenden Kunststoff trennend zu bearbeiten. Es ist jedoch auch möglich, anderen Kunststoff spanend, beispielsweise mit einem Fräser (22) zu bearbeiten.

Zu diesem Zwecke wird ein Werkstück (23) über einer Werkstückplatte (24) an Auslegearmen (25) über nicht dargestellte Spannleiter befestigt. Der Auslegearm (25) führt das mit ihm verbundene Werkstück (23) an dem Bearbeitungswerkzeug (19) vorbei. Er wird dabei von den Motoren (17, 18) in Y- und Z-Richtung gesteuert. Eine Steuerung sowohl in X-Richtung als auch hinsichtlich der Rotation erfolgt durch gesonderte Motore.

Die Steuerung der Bearbeitungsmaschine erfolgt entsprechend Begrenzungslinien (9) der Teilschnitte (12). Diese Begrenzungslinien (9) können mit Hilfe des Cursors umfahren werden. Beim Umfahren der Begrenzungslinien (9) entstehen elektrische Impulse, die zur Steuerung der Bearbeitungsmaschine (5) herangezogen werden. Auf diese Weise wird jedem Teilschnitt (12) entsprechend das Werkstück (23) im Sinne des gewünschten Modelles bearbeitet. Dabei können die Begrenzungslinien (9) des jeweiligen Teilschnittes (12) nicht nur manuell mit Hilfe des Cursors, sondern auch mit Hilfe eines in den Computer einzugebenden Konturfindungsprogrammes abgefahren werden.

Es ist auch möglich, das Werkstück (23) fest auf der Werkstückplatte (24) zu befestigen und das Bearbeitungswerkzeug (19) auf seiner Oberfläche entlang zu führen.

## Patentansprüche

1. Verfahren zur Herstellung eines räumlichen Modells von von außen nicht zugänglichen, sich innerhalb eines Körpers befindlichen Strukturen mit Hilfe computertomographischer Teilschnitte (12), die auf vorgegebenen Ebenen in einer vorgegebenen Reihenfolge durch die Strukturen gelegt werden und deren Begrenzungen auf einen Modellwerkstoff übertragen werden, dadurch gekennzeichnet, daß die Begrenzungslinien (9) der computer-tomographischen Teilschnitte (12) in räumliche Koordinaten elektronisch zur Steuerung mindestens eines Bearbeitungswerkzeuges (19) umgesetzt werden, wobei das Bearbeitungswerkzeug (19) im Bereich zwischen zwei Teilschnitten (12) liegender Abstände mit Hilfe interpolierter Werte gesteuert wird, die vom Rechner aufgrund der auf den Teilschnitten (12) liegenden Koordinaten berechnet werden und ein Werkstück (23) unmittelbar aus einem Block des Modellwerkstoffes hergestellt wird.

2. Verfahren nach Anspruch 1, dadurch gekennzeichnet, daß das Bearbeitungswerkzeug (19) auf gedachten Ebenen, die in ihrer Lage einem jeweiligen Teilschnitt (12) entsprechen, an einem zur Herstellung des Modells geeigneten Werkstück (23) entlanggeführt wird.

3. Verfahren nach Anspruch 1 und 2, dadurch gekennzeichnet, daß die räumlichen Koordinaten eines jeden Teilschnittes (12) in einen Computer (2) eingelesen und in diesem gespeichert werden.

## Claims

1. A method of producing a three-dimensional model of structures which are not accessible from the exterior and which are within a body, by means of computer-tomographic partial sections (12) which are put on predetermined planes in a predetermined sequence through the structures and whose boundaries are transferred on to a model material, characterised in that the boundary lines (9) of the computer-tomographic partial sections (12) are electronically converted into three-dimensional co-ordinates for the control of at least one processing tool (19), wherein the processing tool (19) is controlled in the region of spacings between two partial sections (12) by means of interpolated values which are computed by the computer on the basis of the co-ordinates on the partial sections (12) and a workpiece (23) is produced directly from a block of the model material.

2. A method according to claim 1 characterised in that the processing tool (2) is guided along a workpiece (23) which is suitable for the production of the model on notional planes which correspond in their position to a respective partial section (12).

3. A method according to claim 1 and claim 2 characterised in that the three-dimensional co-ordinates of each partial section (12) are read into a computer (2) and stored therein.

## Revendications

1. Procédé pour la production d'un modèle tridimensionnel de structures inaccessibles de l'extérieur se trouvant à l'intérieur d'un corps à l'aide de coupes partielles (12) tomodensitométriques, qui sont réalisées à travers les structures sur des plans prédéterminés dans un ordre prédéterminé et dont les contours sont transmis à un matériau de modélisation, caractérisé en ce que les lignes de contour (9) des coupes partielles (12) tomodensitométriques sont converties en coordonnées tridimensionnelles pour la commande d'au moins un outil de traitement (19), l'outil de traitement (19) étant commandé dans le secteur des espacements compris entre deux coupes partielles (12) à l'aide de valeurs interpolées, qui sont calculées par l'ordinateur à partir des coordonnées attribuées aux coupes partielles (12) et une pièce usinée (23) est immédiatement produite à partir d'un bloc du matériau de modélisation.

2. Procédé selon la revendication 1, caractérisé en ce que l'outil de traitement (19) est guidé sur des plans fictifs correspondant par leur position à une coupe partielle (12) donnée le long de la pièce usinée (23) appropriée pour la production du modèle.

3. Procédé selon les revendications 1 et 2, caractérisé en ce que les coordonnées spatiales de chaque coupe partielle (12) sont lues dans un ordinateur (2) et enregistrées dans celui-ci.
